# EUROPEAN PATENT SPECIFICATION

(11) **EP 4 536 340 B1**
(45) Date of publication and mention of the grant of the patent: **15.04.2026**
(21) Application number: 23732145.0
(22) Date of filing: 13.06.2023
(51) Int. Cl.: A61N 1/05, A61N 1/36, A61B 5/00, A61N 1/372

(54) **HIGH DENSITY BRAIN ELECTRODE ASSEMBLY FOR READ-OUT AND/OR STIMULATION OF BRAIN TISSUE**
HOCHDICHTE HIRNELEKTRODENANORDNUNG ZUM LESEN UND/ODER STIMULIEREN VON HIRNGEWEBE
ENSEMBLE ÉLECTRODES CÉRÉBRALES HAUTE DENSITÉ POUR L'OBTENTION DE TRACÉS ET/OU LA STIMULATION DU TISSU CÉRÉBRAL

(30) Priority: 13.06.2022 NL 2032152
(43) Date of publication of application: 16.04.2025
(73) Proprietor: Koninklijke Nederlandse Akademie van Wetenschappen, 1011 JV Amsterdam (NL); Phosphoenix B.V., 1105 AZ Amsterdam (NL)
(72) Inventor: ROELFSEMA, Pieter, 1105 BA AMSTERDAM (NL); BALK, Stijn, 1105 BA AMSTERDAM (NL); VAN VELDHUIZEN, John, 1105 BA AMSTERDAM (NL); WANG, Feng, 1105 BA AMSTERDAM (NL); MONNA, Bert, 1105 BA AMSTERDAM (NL); CHEN, Xing, 1105 BA AMSTERDAM (NL); LI, Bingshuo, 1105 BA AMSTERDAM (NL); LOZANO, Antonio, 1105 BA AMSTERDAM (NL); ORLEMANN, Corinne, 1105 BA AMSTERDAM (NL); HEEMSKERK, Maurice, 1105 BA AMSTERDAM (NL); KOOIJMANS, Roxana, 1105 BA AMSTERDAM (NL)
(74) Representative: EP&C
(86) International application number: PCT/EP2023/065850
(87) International publication number: WO 2023/242215

(56) References cited:
- US-A1- 2010 094 382
- US-A1- 2010 114 272
- US-B1- 6 353 762
- US-B1- 7 181 288

## Description

### TECHNICAL FIELD

The present disclosure relates to the field of biomedical engineering and, in particular, to a high density brain electrode assembly, for example a high density brain read-out and/or stimulation electrode assembly to read-out and/or stimulate a lateral geniculate nucleus of the thalamus to create visual perceptions in a mammal. The invention also relates to a neuroprosthetics system for substituting or inducing a missing or impaired sensory functionality of a mammal, by electrical stimulation of electrodes of such high density brain electrode assembly, for example to restore functional vision of a human being suffering from partial or total blindness.

### BACKGROUND OF THE INVENTION

Neuroprosthetics or neural prosthetics, is a technical discipline related to neuroscience and biomedical engineering concerned with the development of neural prostheses. A neural sensory prosthesis, in its most general form, is a device or system designed to substitute or induce, by neural read-out and/or stimulation in a respective region of the cerebral cortex of a mammal, a sensory functionality, such as visual, auditory, tactile, or olfactory functionality, that might have been damaged as a result of an injury or a disease.

In the field of sensory restoration of visual functionality, for example, damage to the visual system can be generally classified into two groups, i.e. a first group with damage in the visual processing pathway up to and including the ganglion cell layer in the retina, and a second group with damage after this processing stage impairing information flow between the retina and the visual cortex.

For the first group of patients, significant progress has been made towards solutions in the form of gene therapy in the retina and retinal prostheses for implantation in the retina, providing low vision to mammals, in which the retinal ganglion cells that connect the eye to the brain have been spared. Retinal implants and gene therapy cannot be used for people with extensive damage to the ganglion cells and/or optic nerve. For the large group of patients whose sight cannot be restored in the retina, i.e. the above-mentioned second group, prostheses comprised of surface electrical contacts for application over the exterior surface of the visual cortex have been developed, as well as prostheses comprising electrodes for activation of neurons located deeper in the visual cortex, allowing the generation of a sparse subset of phosphenes.

WO20043790 A1 discloses a neuroprosthetic system for substituting a sensory modality of a mammal by electrical stimulation of a region of the cerebral cortex of said mammal corresponding to said neural modality to be substituted, said system comprising:
at least one sensor, for use by said mammal, arranged for generating a sensed data feed by sensing a neural modality to be substituted;
an electrode unit, comprised of a plurality of three-dimensional arrays of flexible, elongated electrode shanks, arranged for intracortical implantation for a dense occupation of such region of the cerebral cortex of said mammal arranged for providing functional coverage of the sensory modality, each shaft comprising multiple electrical contacts, for electrical stimulation of subsets of locations in said region of the cerebral cortex;
a rigid electrode support structure, arranged for simultaneously guiding said flexible electrode shanks of an array into said region of the cerebral cortex of said mammal during intracortical implantation, and for retracting said support structure after implantation of an array of flexible electrode shanks;
a driving unit, arranged for electrically driving said electrode unit for stimulating said subsets of locations in said region of the cerebral cortex,
a recording unit, arranged obtaining neural recordings through said electrode unit in said region of the cerebral cortex; and
a processing unit, arranged for analysing said sensed data feed for providing stimulation patterns for electrically driving groups of electrical contacts of said electrode unit corresponding to subsets of locations in said region of the cerebral cortex, for substituting said sensory modality.

The neuroprosthetic system of WO20043790 A1 is based on the insight that a functional substitution of an impaired neural modality necessitates electrical stimulation of dense locations in a respective region of the cerebral cortex providing functional coverage of the sensory modality. This is achieved by providing plural three-dimensional arrays of flexible electrode shanks and a rigid electrode support structure, arranged for inserting in a simultaneous manner (i.e. in one single step in which all electrode shanks of one array are displaced together instead of one-by-one) all flexible electrode shanks of an array into a target region of the cerebral cortex of the mammal, and for retracting the support structure after implantation of the flexible electrode shanks.

In this way, a dense set of very thin flexible electrode shanks for uniform stimulation of locations in the cortex can be applied, in particular in cortical areas that are otherwise difficult to access, such as in a sulcus of the cerebral cortex. In this way, these areas have such a dense distribution of electrodes in said area of the cerebral cortex of the patient or mammal that functional coverage of the sensory modality is achieved.

Another location that is suitable to provide electrical stimulation to create visual perceptions in a mammal to improve or restore functional vision of a mammal suffering from partial or total blindness is a lateral geniculate nucleus (LGN) of the thalamus. Also other non-superficial locations in the brain of a mammal, such as deeper structures of cortex, may be suitable to apply electrical stimulation or to carry out electrical read-outs of the brain tissue.

The electrode unit disclosed WO20043790 A1 is not arranged to stimulate a LGN which is located deeper in the brain and has a smaller volume than the visual cortex. In addition, the implantation of this electrode unit in the brain requires a large surgical impact that involves temporary removal of large parts of the skull of the patient.

US 7 181 288 teaches an assembly as defined in claim 1, without shuttle elements as claimed, however.

There is a need for electrode assemblies for read-out and/or stimulation of the brain that can be used for electrical read-out and/or stimulation of non-superficial brain tissue and that can be implanted with low surgical impact on the patient.

The present invention provides a high density brain electrode assembly for read-out and/or stimulation of brain tissue, comprising
an elongated main shaft comprising a plurality of guiding lumens, extending to a distal end of the main shaft,
a plurality of elongated electrode shanks, each comprising a plurality of electrical contacts, wherein each electrode shank extends through one of the plurality of guiding lumens, and wherein each electrode shank is movable from a retracted position in which the electrode shank is retracted within the main shaft to an extended position in which the electrode shank extends distally from the distal end of the main shaft,
a plurality of shuttle elements, wherein each shuttle element extends through one of the plurality of guiding lumens, and wherein each shuttle element is movable between a retracted position in which the shuttle element is retracted within the distal end of the main shaft and an extended position in which the shuttle element extends distally from the distal end of the main shaft,
wherein the electrode shanks are more flexible than the shuttle elements, wherein each shuttle element is connected with an electrode shank of the plurality of electrode shanks to move the respective electrode shank from the retracted position to the extended position.
wherein the high density brain electrode assembly is arranged to distribute distal ends of the electrode shanks in extended position over a surface area in a plane perpendicular to a longitudinal axis of the main shaft which is larger than a largest cross section of the distal end of the main shaft perpendicular to the longitudinal axis of the main shaft.

The high density brain electrode assembly is constructed to allow read-out and/or stimulation of non-superficial brain tissue, for example deep brain tissue, such as an LGN. The high density brain electrode assembly comprises a main shaft to penetrate the brain to a desired implantation location at a certain depth in the brain. During penetration of the main shaft into the brain the electrode shanks and the shuttle elements are located in the retracted position in which the electrode shanks and the shuttle elements are retracted in the main shaft. When the main shaft is located at the desired implantation location, the shuttle elements and the electrode shanks may be moved from the retracted position to the extended position to locate the electrical contacts of the electrode shank in the tissue of interest, such as a LGN.

The electrode shanks are more flexible than the shuttle element. Each shuttle element is connected to one of the electrode shanks, for example at their respective distal ends, such that the shuttle element can be used to push the electrode shank from the retracted position to the extended position. Once the electrode shank is placed in the extended position, the connection between the shuttle element and the electrode shank may be released to allow the shuttle element to be moved back to the retracted position, while the electrode shank remains in the extended position.

The connection between shuttle element and electrode shank may be a releasable connection. Such releasable connection may be released by an active release step, or as a consequence of the movement of the shuttle element from the extended position to the retracted position. The releasable connection may also be based on a brain fluid dissolvable adhesive, for example a polyethylene glycol. That is, after movement of the electrode shanks to the extended position, the adhesive may dissolve, therewith releasing the connection between the electrode shank and the associated shuttle element to allow safe retraction of the shuttle element to the retracted position. Polyethylene glycol may dissolve, for example, after seconds to minutes after it has come into contact with brain fluid.

In another embodiment, the shuttle elements may be arranged to at least partially lose their stiffness after implantation, for example by using silk fibroins that may have a decreasing stiffness after implantation in the brain. In such embodiment, the shuttle element and the electrode shank may be combined in a single element in which the shuttle element practically forms a releasable connection to the electrode shank that is released by losing its stiffness.

The combination of the shuttle element and the electrode shank enables the placement of the electrode shank into the desired tissue by movement of the combination of shuttle element and electrode shank to the extended position, whereby the shuttle element provides sufficient stiffness for pushability of this combination without the need to provide a relatively stiff electrode shank. The flexibility of the electrode shank after release of the shuttle element, and possibly movement back to the retracted position provides the advantage that a relative movement between the tissue in which the electrode shanks are placed and the tissue or bone that holds the main shaft is not substantially hindered by the stiffness of the electrode shanks, leading to tissue damage and/or gliosis.

The high density brain electrode assembly is arranged to distribute distal ends of the electrode shanks over a surface area in a plane perpendicular to a longitudinal axis of the main shaft which is larger than a largest cross section of the distal end of the main shaft perpendicular to the longitudinal axis of the main shaft.

By this construction, the electrode shanks can be guided towards a non-superficial brain location through a small-diameter bundle of guiding lumens arranged in the main shaft, while at the distal end of the main shaft the electrode shanks fan out, for example in a cone shape, to a larger surface area than the cross section of the distal end of the main shaft. The surface area over which the electrode shanks may be distributed in a direction perpendicular to the longitudinal axis of the main shaft may be at least twice the surface area of the cross section of the distal end of the main shaft, for example at least five times the surface area of the cross section of the distal end of the main shaft. This may result in a distribution of the electric contacts of the electrode shanks over a relatively large volume compared with the cross-section of the distal end of the main shaft. At the same time the actual volume of brain tissue, in which a large number of electrical contacts may be distributed by the electrode shanks may be small, for example 50 mm³ to 250 mm³ resulting in a relative high density of electrical contacts distributed in the respective volume.

In an embodiment, the high density brain electrode assembly is arranged to distribute distal ends of the electrode shanks over a surface area in a plane perpendicular to a longitudinal axis of the main shaft which is larger than a largest cross section of the implantable part of the main shaft perpendicular to the longitudinal axis of the main shaft, for example at least twice the surface area of the cross section of the implantable part of the main shaft, at least five times the surface area of the cross section of the implantable part of the main shaft. The implantable part comprises the part of the main shaft that may extend through the brain tissue after implantation of the main shaft.

In an embodiment, one or more of the plurality of guiding lumens have a non-zero exit angle with respect to a longitudinal axis of the main shaft such that the electrode shank, in the extended position, extending from the respective guiding lumen diverges away from the longitudinal axis. To diverge the electrode shank, in the extended position, away from the longitudinal axis of the main shaft, the exit angle of the guiding lumens at the distal end of the main shaft may be arranged at an angle, i.e. having a non-zero angle with respect to the longitudinal axis of the main shaft.

Distal openings of the guiding lumens that are located farther away from the longitudinal axis of the main shaft may have a larger exit angle than the distal openings of the guiding lumens that are closer to the longitudinal axis to obtain a desired distribution of the electrode shanks in the volume of brain tissue in which they extend. The exit angles of the guiding lumens may be used to vary the density and/or spacing of the electrode shanks in this volume, for example to optimize phosphene coverage in this volume.

The distal openings of the guiding lumens may be arranged in two or more concentric circles concentrically arranged with respect to the longitudinal axis of the main shaft, whereby the distal openings of an inner concentric circle have smaller exit angles with respect to the longitudinal axis of the main shaft compared to the exit angles of the distal openings of an outer concentric circle. Any other suitable configuration may also be applied.

At the distal end of the main shaft a guiding block may be provided, wherein the guiding block forms angled distal openings arranged at the distal ends of the guiding lumens. The guiding block may be made of any suitable material, such as ceramic, metal or polymeric material, and may be constructed by 3D printing. Since the guiding block may be used to fan out the electrode shanks in a volume of brain issue, the guiding block may also be referred to as fountain block.

In an embodiment, the guiding lumens are formed by tubes or tunnels extending from the proximal end to the distal end of the main shaft. A bundle of tubes may be provided to form at least partially the plurality of guiding lumens through which the shuttle elements and electrode shanks extend. At the distal side of the tubes the tubes may be connected to respective guiding lumens arranged in the guiding block. As an alternative, the tubes may be arranged at the desired exit angles.

In an embodiment, one or more of the shuttle elements may at least at a distal part thereof be pre-shaped in a non-straight configuration, and be adapted to assume a straighter configuration upon an application of force thereto. Such shuttle element may be forced in the straighter configuration when arranged in the respective guiding lumen, and assume partially the non-straight configuration when the respective shuttle element is moved to the extended position and at least partially extends from the distal end of the main shaft. This pre-shaped non-straight configuration may facilitate the fanning out of at least a part of the shuttle elements to a larger cross section than the largest cross section of the distal end of the main shaft perpendicular to the longitudinal axis of the main shaft. The pre-shaped shuttle elements with non-straight configuration may be used in combination or as an alternative for the non-zero exit angles of the guiding lumens.

The electrode shanks may be formed as thin strips having at least along the part that extends, in the extended position, in distal direction from the distal end of the main shaft a series of electrical contacts. By arranging the plurality of electrode shanks in the desired non-superficial brain tissue, whereby each electrode shank has a plurality of electrical contacts along the length of the electrode shank, a high density three dimensional array or lattice of electrical contacts is formed that enables effective stimulation of and/or recording from non-superficial, for example deep brain tissue.

In an embodiment, the electrode shanks are strips having a thickness in the range of 0.1 µm to 40 µm, for example in the range of 1 µm to 20 µm. The strips may have a width in the range of 1 µm to 250 µm, for example in the range of 25 µm to 150 µm. The strips may be made of any flexible and tissue-biocompatible material, such as polyimide or SU-8. The electrical contacts may be made of materials that can pass the current into the tissue, for example iridium oxide, microstructured platinum, platinum-iridium, PEDOT, or titanium nitride. Each electrode shank may have for example 5 to 100 electric contacts, such as 10 to 50 electric contacts.

In an embodiment, proximal ends of the electrode shanks are connected to each other. By coupling of the electrode shanks to each other, the electrical leads to each of the electrical contacts can be combined to a single bundle of electrical leads. The electrical leads can be provided using printing or etching techniques or the like, available from semiconductor engineering.

In an embodiment, the electrode shanks may be connected to each other by an electrical lead element. The electrical lead element may provide support for electrical wiring to each of the electrical contacts on the electrode shanks. The electrical leads can be provided using printing or etching techniques or the like, available from semiconductor engineering.

In an embodiment, the electrode shanks and the electrical lead element are cut integrally from a single sheet of material, for example a film material on which the electrical leads are provided.

In an embodiment, the main shaft comprises at least 10, for example at least 20 guiding lumens. The high density brain electrode assembly is constructed for deployment of a plurality of electrode shanks in non-superficial brain tissue.

In an embodiment, each guiding lumen is arranged to guide one electrode shank and one shuttle element. By providing a separate guiding lumen for each combination of electrode shank and shuttle element, the movement of the combination of electrode shank and shuttle element from the retracted position to the extended position and therewith the implantation path of the electrode shank to the extended position can be controlled more accurately.

In an embodiment, the guiding lumens have an inner diameter of 50 µm to 500 µm, for example in the range of 80 µm to 250 µm.

In an embodiment, each electrode shank comprises at or near its distal end an opening, wherein each shuttle element comprises at or near its distal end a tapered end that at least partially extends through the opening to provide the releasable connection between the electrode shank and the shuttle element. Such combination of opening and tapered end provides a simple and effective method to provide a releasable connection that will automatically release when the shuttle element is retracted toward the retracted position.

In an embodiment, an extendable part of the electrode shank extends in the extended position in the range of 0.5 mm to 50 mm from the distal end of the main shaft, for example in the range of 5 mm to 30 mm.

In an embodiment, the electrode assembly comprises an insert element that is connected to proximal ends of the shuttle elements to simultaneously move the shuttle elements between the retracted position and the extended position. The insert element may proximally extend out of the main shaft for manual or mechanical manipulation of the insert element. After movement of the electrode shanks from the retracted position to the extended position, the insert element may be together with the shuttle elements completely removable out of the main shafts, or it may remain in a retracted position. As an alternative to the insert element that is connected to all shuttle elements, each shuttle element may be individually movable between the retracted position and the extended position, and potentially removable out of the main shaft after moving the respective electrode shank to the extended position. Also, multiple insert elements may be provided that are connected to sub-groups of shuttle elements.

In an embodiment, the shuttle elements comprise metal, such as tungsten. The rigidity of the shuttle elements is selected such, that the electrode shanks can be positioned at once, for example by hand force or mechanically driven by a suitable tool, in the desired position in the non-superficial brain tissue.

In an embodiment, the main shaft has a diameter of 0.5 mm to 5 mm. The main shaft is constructed to house a plurality of guiding lumens in each of which there is arranged an electrode shank and a shuttle element. The main shaft may have a length in the range of 1 cm to 20 cm, for example in the range of 3 cm to 12 cm.

The invention further relates to a neuroprosthetic system for substituting a sensory modality of a mammal by electrical stimulation of a non-superficial brain region of said mammal corresponding to said neural modality to be substituted, said system comprising:
at least one sensor, for use by the mammal, arranged for generating sensed data feed by sensing a neural modality to be substituted,
at least one high density brain electrode assembly as claimed in any of the claims 1-13,
a driving unit, arranged for electrically driving the electrical contacts of the high density brain electrode assembly for stimulating said non-superficial brain region, and
a processing unit arranged for analysing the sensed data feed for providing stimulation patterns for electrically driving the electrical contacts of the high density brain electrode assembly corresponding to subsets of locations in said non-superficial brain region, for substituting said sensory modality.

In an embodiment, the neuroprosthetic system is arranged for substituting visual perception, in a non-superficial brain region of a mammal, wherein said at least one sensor comprises at least one portable imaging unit arranged for capturing images and generating a captured image data feed.

In an embodiment, the system may comprise a switching device for channelling one or more stimulation signals of said driving unit to subsets of electrical contacts located within said non-superficial brain region providing said functional coverage of the sensory modality. The switching device enables the system to connect multiple (groups) of electrode shanks or particular electrical contacts thereof to one single signal generator. As such, it is not necessary to have a signal generator for each electrical contact, and only a significant lower amount required which preferably corresponds to the number of different stimulation signals the driving unit is required to generate.

The present disclosure further relates to a method for substituting a sensory modality of a mammal by electrical stimulation of a non-superficial brain region of said mammal using at least one high density brain electrode assembly as claimed in any of the claims 1-13, the method not constituting a portion of the claimed invention and comprising the steps:
implanting the main shaft of the high density brain electrode assembly at a desired implantation location;
moving the plurality of shuttle elements from the retracted position to the extended position to move the respective electrode shanks from the retracted position to the extended position to position the electrical contacts in their implanted positions,
sensing with a sensor a neural modality to be substituted for generating sensed data,
electrically driving the electrical contacts of the high density brain electrode assembly for stimulating said non-superficial brain region, and
analysing the sensed data feed for providing stimulation patterns for electrically driving the electrical contacts of the high density brain electrode assembly corresponding to subsets of locations in said non-superficial brain region, for substituting said sensory modality.

### BRIEF DESCRIPTION OF THE DRAWINGS

Fig. 1 depicts, schematically, an embodiment of a high density brain electrode assembly before implantation;
Fig. 2 depicts, schematically, the high density brain electrode assembly of Figure 1 after deployment of the electrode shanks;
Fig. 3 depicts, schematically, a sectional view of the high density brain electrode assembly of Figures 1 and 2 before implantation;
Fig. 4 depicts, schematically a cross section view of the main shaft at line B-B shown in Figure 3;
Figs. 5A-5C depict the steps of deployment of an electrode shank of a high density brain electrode assembly;
Fig. 6 depicts, schematically, a sectional view of an alternative embodiment of a high density brain electrode assembly; and
Fig. 7 depicts the high density brain electrode assembly implanted in the brain for stimulation of the LGN of a human.

### DETAILED DESCRIPTION

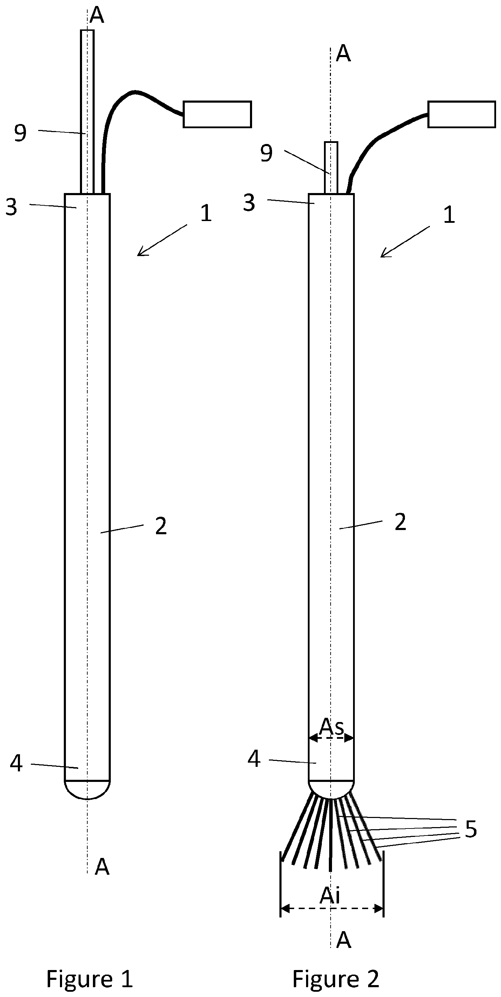

Figure 1 schematically depicts a high density brain electrode assembly 1 for read-out and/or stimulation of non-superficial brain tissue. The electrode assembly 1 is arranged to be implanted in a brain of a mammal to arrange a plurality of electrode shanks each having a plurality of electric contacts in a non-superficial brain region of interest, in particular a deep brain region of interest, for example a lateral geniculate nucleus (LGN) of the thalamus of a human, to provide electric read-out and/or stimulation in the respective non-superficial brain region. The electrode assembly 1 comprises an elongated main shaft 2 having a proximal end 3 and a distal end 4. In the main shaft 2 a plurality of electrode shanks are arranged in a retracted position, in which the electrode shanks are mainly accommodated in the main shaft 2 and do not extend beyond the distal end 4 of main shaft 2.

Figure 2 schematically depicts the electrode assembly 1 of Figure 1 in an implanted state. The electrode shanks 5 are extended to the extended position in which the electrode shanks 5 extend from the distal end 4 of the main shaft 2. In implanted state, the electrical contacts of the electrode shanks 5 are arranged to be in contact with the tissue in which the electrode shanks 5 are introduced. The electrode shanks 5 are embodied as flexible strips such that relative movements within a certain range of movement between the main shaft 2 and the electric contacts on the electrode shanks 5 can be accommodated. Thus, the electrode shanks 5 are relatively free to move with respect to the main shaft 2 therewith allowing the electrode shanks 5 to follow movements of the tissue in which they are implanted without the need of the main shaft to also follow the movements of the electrode shanks 5. The use of flexible electrode shanks 5 however makes the implantation of the electrode shanks 5 through the tissue in which they are implanted more difficult. To effectively implant the electrode shanks 2 to the desired location shuttle elements are used, as will be described hereinafter.

The electrode assembly 1 is arranged to distribute the distal ends of the electrode shanks over a surface area Ai in a plane perpendicular to a longitudinal axis A-A of the main shaft which is larger than a largest cross section As of the main shaft 2 perpendicular to the longitudinal axis A-A of the main shaft 2. Thus, the electrode shanks 5 fan out of the distal end 4 of the main shaft 2 to the surface area Ai which is larger than the cross section As of the main shaft 2. The surface area Ai over which the electrode shanks 5 are distributed may be at least twice the surface area As of the cross section of the main shaft 2, for example at least five times the surface area As. As a result, the electrode shanks 5 extend in a cone shaped volume, wherein electric contacts provided on the electrode shanks create a 3D lattice of electric contacts in the tissue in which the electrode shanks 5 are implanted.

The extendable part of the electrode shanks 5, i.e. the part that extend in the extended position from the distal end 4 of the main shaft may have a length in the range of 0.5 mm to 30 mm, for example between 10 mm and 20 mm. The diameter of the main shaft 2 may for example be in the range of 1 mm to 10 mm. The dimensions may also depend on the application. For example, an electrode assembly for mice may have substantial smaller dimensions than an electrode assembly for humans.

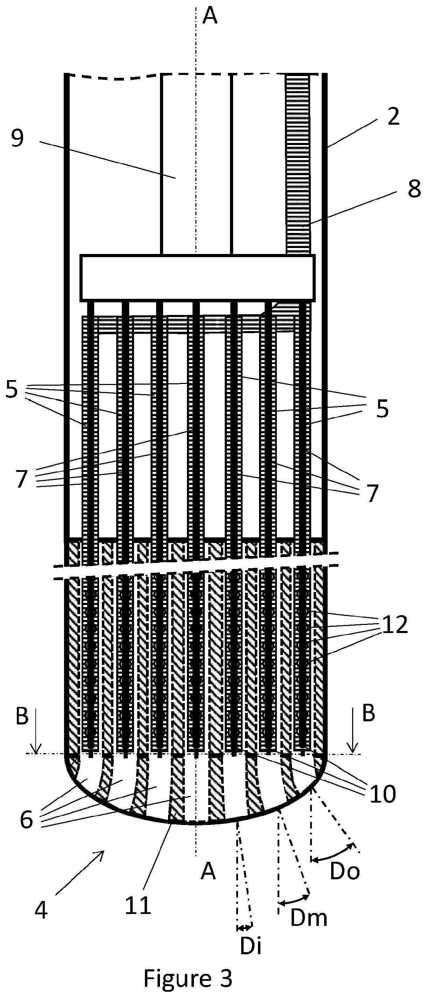

Figure 3 shows a sectional view of the distal section of the electrode assembly 1. The main shaft 2 comprises a plurality of guiding lumens 6 that extend mainly in longitudinal direction of the main shaft, i.e. parallel to the longitudinal axis A-A of the main shaft 2. In each of the guiding lumens 6, an electrode shank 5 and a shuttle element 7 is provided.

The shuttle elements 7 are elongated elements, for instance wires made of metal, such as tungsten, silicon or another suitable material. The shuttle elements 7 are less flexible than the electrode shanks 5. The shuttle elements 7 are movable between a retracted position, as shown in Figure 3 and an extended position in which the shuttle elements extend at least partially from distal openings of the guiding lumens 6.

The electrode shanks 5 are at their proximal ends connected to each other by an electrical lead element 8. The electrode shanks 5 and the electrical lead element 8 may be cut as an integral part from a sheet of material, for example a film material on which the electrical leads are provided. The electrical leads can be made using printing or etching techniques or the like. The electrical lead element 8 may be rolled or folded to create a compact format.

In the embodiment of Figure 3, the shuttle elements 7 are at their proximal ends connected to an insert element 9. The insert element 9 facilitates simultaneous movement of the shuttle elements 7 between the retracted position and the extended position. The insert element 9 extends proximally from the proximal end 3 of the main shaft 2 (see figures 1 and 2) to allow manipulation of the insert element 9. In an alternative embodiment, a separate insert element may be provided for each shuttle element 7 to facilitate individual movement of the shuttle elements 7. It is also possible to provide two or more insert elements each connected to a sub-group of shuttle elements 7 to facilitate movement of the shuttle elements per sub-group.

The distal ends of the electrode shanks 5 are releasably connected to the distal ends of the shuttle elements 7 by means of a releasable connection 10. In the shown embodiment, the releasable connection 10 is formed by a tapered part at the distal end of the shuttle element 7 that extends through an opening in the distal end of the electrode shank 5.

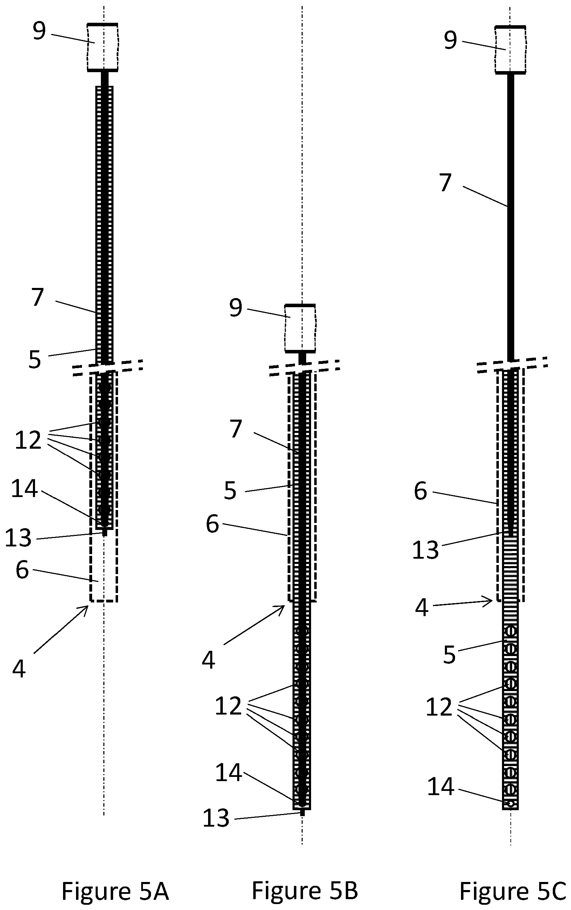

As shown in Figures 5A-5C, the releasable connection 10 is provided such that movement of the shuttle elements 7 from the retracted position to the extended position will take along the electrode shanks 5 from the retracted position to the extended position. In the extended position, the releasable connection 10 may be released and the shuttle elements 7 may be moved back to the retracted position, while the electrode shanks 5 remain in the extended position.

At the distal end 4 of the main shaft 2, a guiding block 11 is provided. The guiding block 11 comprises at least distal parts of the guiding lumens 6 and form distal openings at the distal end 4 of the main shaft 2.

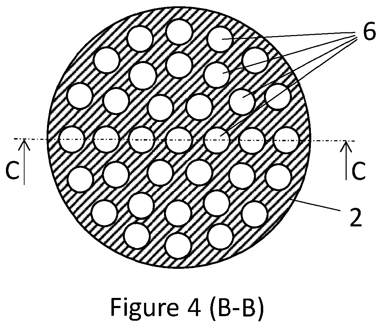

Figure 4 shows a cross-section B-B of the main shaft 2 showing an example of the distribution of guiding lumens 6 over the cross-section of the main shaft. In this embodiment, the main shaft 2 comprises 35 guiding lumens 6 each arranged for guiding one combination of electrode shank 5 and shuttle element 7. The guiding lumens end in the guiding block 11. The number of guiding lumens may depend on the application. In practice, for human applications, the number of guiding lumens may be at least 10, for example at least 20 up to 50 or more guiding lumens, each guiding lumen 6 accommodating an electrode shank 5 and a shuttle element 7. The guiding lumens 6 may have an inner diameter in the range of 50 µm to 500 µm, for example in the range of 80 µm to 250 µm.

The guiding lumens 6 comprise one central guiding lumen 6 arranged on the longitudinal axis A-A of the main shaft 2. The other guiding lumens 6 are arranged in three concentric circles around the central guiding lumen 6. The three concentric circles comprise an inner circle with six guiding lumens 6, a middle circle with twelve guiding lumens 6 and an outer circle with sixteen guiding lumens 6.

As can be seen in Figure 3, the exit angle of the central guiding lumen 6 is zero, i.e. the direction of extension is parallel to the longitudinal axis A-A. The other guiding lumens 6 have a non-zero exit angle with respect to the longitudinal axis A-A of the main shaft 2. In particular, as shown in Figure 3, the distal openings in the inner circle have a first exit angle Di, the distal openings in the middle circle have a second exit angle Dm and the distal openings in the outer circle have a third exit angle Do, wherein the third exit angle Do is larger than the second exit angle Dm and the second exit angle Dm is larger than the first exit angle Di.

The exit angles Di, Dm, Do of the distal openings facilitate that the shuttle elements 7 and therewith the electrode shanks 5 will extend angled out of the distal end of the main shaft 2. This will create the fanned out cone shaped configuration of the electrode shanks 5 after implantation as shown in Figure 2.

In the shown embodiment all exit angles of one circle, either inner, middle or outer circle will be the same, i.e. first exit angle Di for the inner circle, second exit angle Dm for the middle circle and third exit angle Do for the outer circle. Generally, it may be advantageous to provide larger exit angles for distal openings arranged further from the longitudinal axis A-A to obtain a proper distribution of electrical contacts in the tissue in which the electrode shanks 5 are implanted.

In other embodiments, also other configurations are possible in which exit angles of each distal opening may vary, or wherein the distal openings are not arranged in two or more concentric circles, e.g. any configuration that is configured to fan out at least part of the distal ends of the electrode shanks 5 over a surface area larger than the cross-section of the main shaft 2 may be used.

One or more of the shuttle elements 7 may at least at a distal part thereof be pre-shaped in a non-straight configuration, and be adapted to assume a straighter configuration upon an application of force thereto. This pre-shaped non-straight configuration of the shuttle elements 7 may facilitate the fanning out of the one or more shuttle elements 7 to a larger cross section Ai than the largest cross section As of the distal end 4 of the main shaft 2. The pre-shaped shuttle elements 7 with non-straight configuration may be used in combination or as an alternative for the non-zero exit angles of the guiding lumens 6.

Figures 5A to 5C show, schematically, the steps of implantation of a single electrode shank 5 having electrical contacts 12 into an implanted position.

When the insert element 9 is connected to multiple shuttle elements 7, as shown in Figure 3, all these shuttle elements 7 will be moved simultaneously by movement of the insert element 9. For simplicity, only one guiding lumen 6 with one electrode shank 5 and one shuttle element 7 is shown in Figures 5A-5C.

Figure 5A shows the electrode shank 5 and the shuttle element 7 in the retracted position. In this retracted position shown in Figure 5A, the electrode shank 5 and the shuttle element 7 do not extend beyond the distal end 4 of the main shaft 2.

When the main shaft 2 is located in a suitable implantation location in the brain, the shuttle element 7 can be moved from the retracted position to the extended position by moving the insert element 9 towards the distal end 4 of the main shaft. This movement of the shuttle element 7 will move the electrode shank 5 due to the releasable connection 10 at the distal ends of the electrode shank 5 and the shuttle element 7. The releasable connection 10 is formed by a tapered part 13 at the distal end of the shuttle element 7 that extends through an opening 14 in the distal end of the electrode shank 5. This releasable connection 10 will automatically release when the shuttle element 7 is moved back to the retracted position.

Figure 5B shows the electrode shank 5 and the shuttle element 7 in the extended position in which the extendable part with the electrical contacts 12 extends from the distal end 4 of the main shaft 2. The distal opening of the guiding lumen 6 shown in Figures 5A-5C has a zero exit angle and, as a result, the electrode shank 5 and the shuttle element 7 extend parallel to the longitudinal axis A-A of the main shaft. If the exit angle is non-zero, the extended part of the electrode shank will extend along an angled path with respect to the longitudinal axis of the main shaft.

When the electrode shank 5 is in the extended position, the shuttle element 7 may be moved back to the retracted position by manipulation of the insert element 9. To allow this movement, without pulling back the electrode shank 5, the releasable connection 10 between the electrode shank 5 and the shuttle element 7 should be released.

Figure 5C shows the shuttle element 7 back in the retracted position, while the electrode shank 5 remained in the extended position. The insert element 9 and the shuttle elements 7 connected to the insert element 9 may be partially or completely taken out of the main shaft 2 since, at least in the shown embodiment, they are only used for implantation of the electrode shanks 5 into the desired brain tissue.

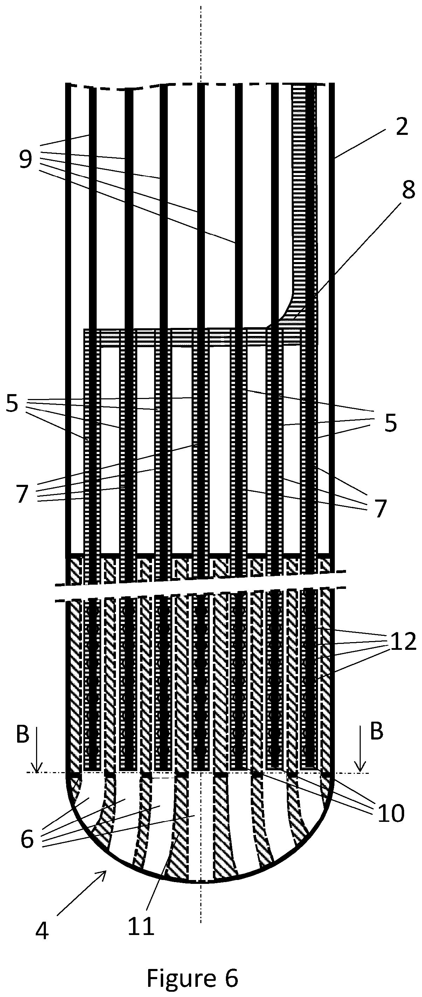

Figure 6 shows a cross section of an alternative embodiment of a high density brain electrode assembly for read-out and/or stimulation of non-superficial brain tissue. In this embodiment, each shuttle element 7 comprises a separate insert element 9 to facilitate individual movement of each of the shuttle elements 7 between the retracted position and the extended position. Further, the releasable connection 10 between the electrode shanks 5 and the shuttle elements 7 is formed by a brain fluid dissolvable adhesive, for example a polyethylene glycol. After movement of the electrode shanks 5 to the extended position, the adhesive may dissolve, therewith releasing the connection between the electrode shank 5 and the associated shuttle element 7 to allow safe retraction of the shuttle element to the retracted position.

In another embodiment, the shuttle elements 7 may be arranged to at least partially lose their stiffness after implantation, for example by using silk fibroins that may have a decreasing stiffness after implantation in the brain. In such embodiment, the shuttle element 7 and the electrode shank 5 may be combined in a single element in which the shuttle element 7 practically releases the electrode shank 5 by losing its stiffness.

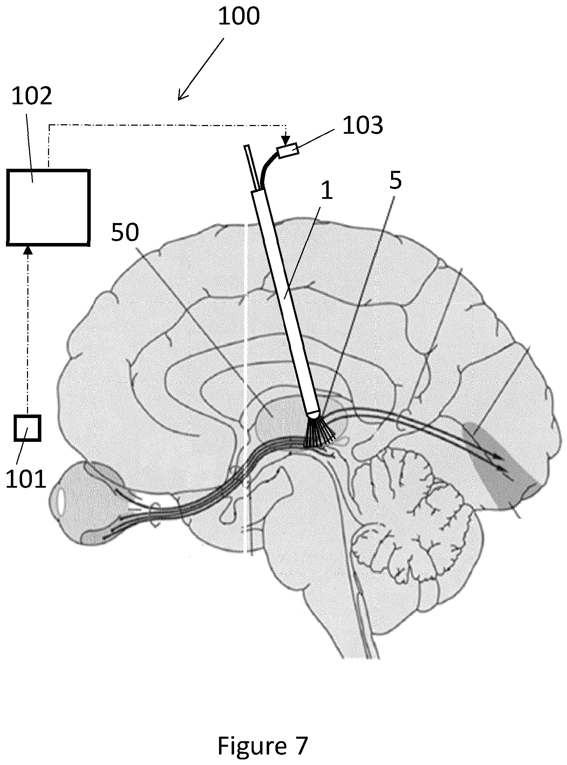

Figure 7 shows the electrode assembly 1 of Figures 1 and 2 implanted in the LGN of a thalamus 50 of a human brain. The electrode assembly 1 can be used as stimulation electrode 1 in a neuroprosthetic system 100 for substituting a sensory modality of a patient, by electrical stimulation of a non-superficial region of the brain of the patient which corresponds to the neural modality that is to be substituted.

More specifically, the neuroprosthetic system 100 is arranged for substituting visual perception in a deep brain region of said mammal by providing electrical stimulation in the LGN in which the electrode shanks 5 extend. For each LGN a high density brain electrode assembly 1 may be implanted in the respective LGN.

The neuroprosthetic system 100 may further comprise at least one sensor 101, a processing unit 102 and a driving unit 103. The at least one sensor 101 comprises at least one portable imaging unit arranged for capturing images and generating a captured image data feed.

The captured image data feed may be fed as a sensed data feed to the processing unit 102. The processing unit 102 is arranged for analysing the sensed data feed for providing stimulation patterns for electrically driving the electrodes of said electrode unit corresponding to subsets of locations in said deep brain region, for substituting said sensory modality. The driving unit 103 is arranged for actually electrically driving the electrical contacts of the deep brain stimulation electrode assembly 1 for stimulating respective stimulation locations in the LGN.

## Claims

1. A high density brain electrode assembly, comprising
an elongated main shaft comprising a plurality of guiding lumens, extending to a distal end of the main shaft,
a plurality of elongated electrode shanks, each comprising a plurality of electrical contacts, wherein each electrode shank extends through one of the plurality of guiding lumens, and wherein each electrode shank is movable from a retracted position in which the electrode shank is retracted within the main shaft to an extended position in which the electrode shank extends distally from the distal end of the main shaft,
a plurality of shuttle elements, wherein each shuttle element extends through one of the plurality of guiding lumens, and wherein each shuttle element is movable between a retracted position in which the shuttle element is retracted within the distal end of the main shaft and an extended position in which the shuttle element extends distally from the distal end of the main shaft,
wherein the electrode shanks are more flexible than the shuttle elements, wherein each shuttle element is connected with an electrode shank of the plurality of electrode shanks to move the respective electrode shank from the retracted position to the extended position, and
wherein the high density brain electrode assembly is arranged to distribute distal ends of the electrode shanks over a surface area in a plane perpendicular to a longitudinal axis of the main shaft which is larger than a largest cross section of the distal end of the main shaft perpendicular to the longitudinal axis of the main shaft.

2. The high density brain electrode assembly of claim 1, wherein one or more of the plurality of guiding lumens have a non-zero exit angle with respect to a longitudinal axis of the main shaft such that the electrode shanks, in the extended position, extending from the one or more guiding lumens diverge away from the longitudinal axis.

3. The high density brain electrode assembly of any of the preceding claims, wherein the guiding lumens are at least partially formed by guiding tubes or tunnels extending through the main shaft.

4. The high density brain electrode assembly of any of the preceding claims, wherein proximal ends of the electrode shanks are connected to each other by an electrical lead element.

5. The high density brain electrode assembly of claim 4, wherein the electrode shanks and the electrical lead element are cut integrally from a single sheet of material.

6. The high density brain electrode assembly of any of the preceding claims, wherein each guiding lumen guides one electrode shank and one shuttle element.

7. The high density brain electrode assembly of any of the preceding claims, wherein the main shaft comprises at least 10 guiding lumens.

8. The high density brain electrode assembly of any of the preceding claims, wherein the guiding lumens have an inner diameter of 50 µm to 500 µm.

9. The high density brain electrode assembly of any of the preceding claims, wherein a distal end of each shuttle element is releasably coupled to a distal end of each associated electrode shank.

10. The high density brain electrode assembly of any of the claims 1-8, wherein each shuttle element is arranged to lose its stiffness at least partially after implantation.

11. The high density brain electrode assembly of any of the preceding claims, wherein an extendable part of each electrode shank extends in the extended position at least 0.5 mm from the distal end of the main shaft.

12. The high density brain electrode assembly of any of the preceding claims, wherein the electrode assembly comprises an insert element that is connected to proximal ends of the shuttle elements to simultaneously move the shuttle elements between the retracted position and the extended position.

13. The high density brain electrode assembly of any of the preceding claims, wherein the main shaft has a diameter of 0.2 mm to 10 mm.

14. A neuroprosthetic system for substituting a sensory modality of a mammal by electrical stimulation of a non-superficial brain region of said mammal corresponding to said neural modality to be substituted, said system comprising:
at least one sensor, for use by the mammal, arranged for generating sensed data feed by sensing a neural modality to be substituted,
at least one high density brain electrode assembly as claimed in any of the preceding claims,
a driving unit, arranged for electrically driving the electrical contacts of the high density brain electrode assembly for stimulation of said non-superficial brain region, and
a processing unit arranged for analysing the sensed data feed for providing stimulation patterns for electrically driving the electrical contacts of the high density brain electrode assembly corresponding to subsets of locations in said non-superficial brain region, for substituting said sensory modality.

15. The neuroprosthetic system according to claim 14, arranged for substituting visual perception in the non-superficial brain region of said mammal, wherein said at least one sensor comprises at least one portable imaging unit arranged for capturing images and generating a captured image data feed.

## Patentansprüche

1. Hochdichte Hirnelektrodenanordnung, umfassend
einen länglichen Hauptschaft, umfassend mehrere Führungslumen, die sich zu einem distalen Ende des Hauptschaftes erstrecken,
mehrere längliche Elektrodenschäfte, die jeweils mehrere elektrische Kontakte umfassen, wobei sich jeder Elektrodenschaft durch eines der mehreren Führungslumen erstreckt und wobei jeder Elektrodenschaft aus einer eingefahrenen Position, in welcher der Elektrodenschaft in dem Hauptschaft zurückgezogen ist, in eine ausgefahrene Position, in welcher sich der Elektrodenschaft distal von dem distalen Ende des Hauptschaftes erstreckt, bewegt werden kann,
mehrere Pendelelemente, wobei sich jedes Pendelelement durch eines der mehreren Führungslumen erstreckt und wobei jedes Pendelelement zwischen einer eingefahrenen Position, in welcher das Pendelelement in dem distalen Ende des Hauptschaftes zurückgezogen ist, und einer ausgefahrenen Position, in welcher sich das Pendelelement distal von dem distalen Ende des Hauptschaftes erstreckt, bewegt werden kann,
wobei die Elektrodenschäfte flexibler als die Pendelelemente sind, wobei jedes Pendelelement mit einem Elektrodenschaft der mehreren Elektrodenschäfte verbunden ist, um den jeweiligen Elektrodenschaft von der eingefahrenen Position in die ausgefahrene Position zu bewegen, und
wobei die hochdichte Hirnelektrodenanordnung dazu ausgelegt ist, distale Enden der Elektrodenschäfte über einen Oberflächenbereich in einer senkrecht zu einer Längsachse des Hauptschaftes verlaufenden Ebene zu verteilen, der größer als ein größter Querschnitt des distalen Endes des Hauptschaftes senkrecht zur Längsachse des Hauptschaftes ist.

2. Hochdichte Hirnelektrodenanordnung nach Anspruch 1, wobei eines oder mehrere der mehreren Führungslumen einen Austrittswinkel ungleich Null in Bezug auf eine Längsachse des Hauptschaftes aufweisen, sodass die Elektrodenschäfte, in der ausgefahrenen Position, sich erstreckend von den ein oder mehreren Führungslumen, von der Längsachse weggehen.

3. Hochdichte Hirnelektrodenanordnung nach einem der vorstehenden Ansprüche, wobei die Führungslumen wenigstens teilweise durch Führungsröhren oder Tunnel gebildet werden, die sich durch den Hauptschaft erstrecken.

4. Hochdichte Hirnelektrodenanordnung nach einem der vorstehenden Ansprüche, wobei proximale Enden der Elektrodenschäfte durch ein elektrisches Leitungselement miteinander verbunden sind.

5. Hochdichte Hirnelektrodenanordnung nach Anspruch 4, wobei die Elektrodenschäfte und das elektrische Leitungselement integral aus einem einzelnen Materialbogen geschnitten sind.

6. Hochdichte Hirnelektrodenanordnung nach einem der vorstehenden Ansprüche, wobei jedes Führungslumen einen Elektrodenschaft und ein Pendelelement führt.

7. Hochdichte Hirnelektrodenanordnung nach einem der vorstehenden Ansprüche, wobei der Hauptschaft wenigstens 10 Führungslumen umfasst.

8. Hochdichte Hirnelektrodenanordnung nach einem der vorstehenden Ansprüche, wobei die Führungslumen einen Innendurchmesser von 50 µm bis 500 µm aufweisen.

9. Hochdichte Hirnelektrodenanordnung nach einem der vorstehenden Ansprüche, wobei ein distales Ende jedes Pendelelements lösbar an ein distales Ende jedes verknüpften Elektrodenschaftes gekoppelt ist.

10. Hochdichte Hirnelektrodenanordnung nach einem der Ansprüche 1-8, wobei jedes Pendelelement dazu ausgelegt ist, seine Steifheit wenigstens teilweise nach der Implantation zu verlieren.

11. Hochdichte Hirnelektrodenanordnung nach einem der vorstehenden Ansprüche, wobei sich ein ausfahrbarer Teil jedes Elektrodenschaftes in der ausgefahrenen Position wenigstens 0,5 mm von dem distalen Ende des Hauptschaft erstreckt.

12. Hochdichte Hirnelektrodenanordnung nach einem der vorstehenden Ansprüche, wobei die Elektrodenanordnung ein Einsatzelement umfasst, das mit proximalen Enden der Pendelelemente verbunden ist, um die Pendelelemente gleichzeitig zwischen der eingefahrenen Position und der ausgefahrenen Position zu bewegen.

13. Hochdichte Hirnelektrodenanordnung nach einem der vorstehenden Ansprüche, wobei der Hauptschaft einen Durchmesser von 0,2 mm bis 10 mm aufweist.

14. Neuroprosthetisches System zum Ersetzen einer sensorischen Modalität eines Säugers durch elektrische Stimulation einer nicht-oberflächlichen Hirnregion des Säugers entsprechend der zu ersetzenden neuronalen Modalität, wobei das System umfasst:
wenigstens einen Sensor, zur Verwendung durch den Säuger, der dazu ausgelegt ist, eine erfasste Dateneinspeisung durch Erfassen einer zu ersetzenden neuronalen Modalität zu erzeugen,
wenigstens eine hochdichte Hirnelektrodenanordnung nach einem der vorstehenden Ansprüche,
eine Ansteuerungseinheit, die dazu ausgelegt ist, die elektrischen Kontakte der hochdichten Hirnelektrodenanordnung zur Stimulation der nicht-oberflächlichen Hirnregion elektrisch anzusteuern, und
eine Verarbeitungseinheit, die dazu ausgelegt ist, die erfasste Dateneinspeisung zu analysieren, um Stimulationsmuster zum elektrischen Ansteuern der elektrischen Kontakte der hochdichten Hirnelektrodenanordnung bereitzustellen, die Teilmengen von Orten in der nicht-oberflächlichen Hirnregion entsprechen, um die sensorische Modalität zu ersetzen.

15. Neuroprosthetisches System gemäß Anspruch 14, das dazu ausgelegt ist, eine visuelle Wahrnehmung in der nicht-oberflächlichen Hirnregion des Säugers zu ersetzen, wobei der wenigstens eine Sensor wenigstens eine tragbare Bildgebungseinheit umfasst, die dazu ausgelegt ist, Bilder zu erfassen und eine Dateneinspeisung aus erfassten Bildern zu erzeugen.

## Revendications

1. Ensemble d'électrodes cérébrales haute densité comprenant
une tige principale allongée comprenant une pluralité de lumières de guidage, s'étendant jusqu'à une extrémité distale de la tige principale,
une pluralité de tiges d'électrode allongées, comprenant chacune une pluralité de contacts électriques, chaque tige d'électrode s'étendant à travers l'une de la pluralité de lumières de guidage, et chaque tige d'électrode étant mobile d'une position rétractée dans laquelle la tige d'électrode est rétractée à l'intérieur de la tige principale à une position déployée dans laquelle la tige d'électrode s'étend distalement à partir de l'extrémité distale de la tige principale,
une pluralité d'éléments de navette, chaque élément de navette s'étendant à travers l'une de la pluralité de lumières de guidage, et chaque élément de navette étant mobile entre une position rétractée dans laquelle l'élément de navette est rétracté à l'intérieur de l'extrémité distale de la tige principale et une position déployée dans laquelle l'élément de navette s'étend distalement à partir de l'extrémité distale de la tige principale,
les tiges d'électrode étant plus flexibles que les éléments de navette, chaque élément de navette étant connecté à une tige d'électrode de la pluralité de tiges d'électrode pour déplacer la tige d'électrode respective de la position rétractée à la position déployée, et
l'ensemble d'électrodes cérébrales haute densité étant agencé pour répartir les extrémités distales des tiges d'électrode sur une zone de surface dans un plan perpendiculaire à un axe longitudinal de la tige principale qui est plus grande qu'une section transversale la plus grande de l'extrémité distale de la tige principale perpendiculaire à l'axe longitudinal de la tige principale.

2. Ensemble d'électrodes cérébrales haute densité selon la revendication 1, une ou plusieurs lumières de la pluralité de lumières de guidage ayant un angle de sortie non nul par rapport à un axe longitudinal de la tige principale de telle sorte que les tiges d'électrode, dans la position déployée, s'étendant à partir de la ou des lumières de guidage divergent en s'éloignant de l'axe longitudinal.

3. Ensemble d'électrodes cérébrales haute densité selon l'une quelconque des revendications précédentes, les lumières de guidage étant au moins partiellement formées par des tubes de guidage ou des tunnels s'étendant à travers la tige principale.

4. Ensemble d'électrodes cérébrales haute densité selon l'une quelconque des revendications précédentes, les extrémités proximales des tiges d'électrode étant connectées l'une à l'autre par un élément de conducteur électrique.

5. Ensemble d'électrodes cérébrales haute densité selon la revendication 4, les tiges d'électrode et l'élément conducteur électrique étant découpés d'un seul tenant à partir d'une feuille unique de matériau.

6. Ensemble d'électrodes cérébrales haute densité selon l'une quelconque des revendications précédentes, chaque lumière de guidage guidant une tige d'électrode et un élément de navette.

7. Ensemble d'électrodes cérébrales haute densité selon l'une quelconque des revendications précédentes, la tige principale comprenant au moins 10 lumières de guidage.

8. Ensemble d'électrodes cérébrales haute densité selon l'une quelconque des revendications précédentes, les lumières de guidage ayant un diamètre intérieur de 50 µm à 500 µm.

9. Ensemble d'électrodes cérébrales haute densité selon l'une quelconque des revendications précédentes, une extrémité distale de chaque élément de navette étant couplée de manière libérable à une extrémité distale de chaque tige d'électrode associée.

10. Ensemble d'électrodes cérébrales haute densité selon l'une quelconque des revendications 1 à 8, chaque élément de navette étant agencé pour perdre sa rigidité au moins partiellement après implantation.

11. Ensemble d'électrodes cérébrales haute densité selon l'une quelconque des revendications précédentes, une partie extensible de chaque tige d'électrode s'étendant dans la position déployée à au moins 0,5 mm de l'extrémité distale de la tige principale.

12. Ensemble d'électrodes cérébrales haute densité selon l'une quelconque des revendications précédentes, l'ensemble d'électrodes comprenant un élément d'insertion qui est connecté aux extrémités proximales des éléments de navette pour déplacer simultanément les éléments de navette entre la position rétractée et la position déployée.

13. Ensemble d'électrodes cérébrales haute densité selon l'une quelconque des revendications précédentes, la tige principale ayant un diamètre de 0,2 mm à 10 mm.

14. Système neuroprothétique destiné à substituer une modalité sensorielle d'un mammifère par stimulation électrique d'une région cérébrale non superficielle dudit mammifère correspondant à ladite modalité sensorielle à substituer, ledit système comprenant :
au moins un capteur, destiné à être utilisé par le mammifère, agencé pour générer un flux de données détectées en détectant une modalité sensorielle à remplacer,
au moins un ensemble d'électrodes cérébrales haute densité selon l'une quelconque des revendications précédentes,
une unité de commande, agencée pour commander électriquement les contacts électriques de l'ensemble d'électrodes cérébrales haute densité pour stimuler ladite région cérébrale non superficielle, et
une unité de traitement agencée pour analyser le flux de données détectées afin de fournir des motifs de stimulation pour commander électriquement les contacts électriques de l'ensemble d'électrodes cérébrales haute densité correspondant à des sous-ensembles d'emplacements dans ladite région cérébrale non superficielle, afin de substituer ladite modalité sensorielle.

15. Système neuroprothétique selon la revendication 14, agencé pour substituer la perception visuelle dans la région cérébrale non superficielle dudit mammifère, ledit au moins un capteur comprenant au moins une unité d'imagerie portable agencée pour capturer des images et générer un flux de données d'image capturées.
